# EUROPEAN PATENT SPECIFICATION

(11) **EP 1 365 711 B1**
(45) Date of publication and mention of the grant of the patent: **09.04.2008**
(21) Application number: 02748352.8
(22) Date of filing: 11.02.2002
(51) Int. Cl.: A61F 2/30

(54) **ORTHOPEDIC IMPLANTS HAVING ORDERED MICROGEOMETRIC SURFACE PATTERNS**
ORTHOPÄDISCHE IMPLANTATE MIT GEORDNETEN MIKROGEOMETRISCHEN OBERFLÄCHENMUSTERN
IMPLANTS ORTHOPEDIQUES PRESENTANT DES MOTIFS SUPERFICIELS MICROGEOMETRIQUES ORDONNES

(30) Priority: 16.02.2001 US 784284
(43) Date of publication of application: 03.12.2003
(73) Proprietor: BIOLOK International, Inc., Deerfield Beach, Florida 33442 (US)
(72) Inventor: RICCI, John, Middletown, NJ 07748 (US); ALEXANDER, Harold, Short Hills, NJ 07078 (US); NAIMAN, Charles DI, deceased (US); HOLLANDER, Bruce, L., Deerfield Beach, FL 33442 (US); KOZAK, Ingo, Deerfield Beach, FL 33442 (US)
(74) Representative: Lorenz, Werner
(86) International application number: PCT/US2002/004093
(87) International publication number: WO 2002/069851

(56) References cited:
- WO-A-01/58374
- WO-A-95/12369
- US-A- 4 330 891
- US-A- 4 553 272
- US-A- 4 608 052
- US-A- 4 752 294
- US-A- 4 778 469
- US-A- 5 002 572
- US-A- 5 108 434
- US-A- 5 246 530
- US-A- 5 607 607
- US-A- 5 716 412
- US-A- 5 989 027
- US-A1- 2001 039 454

## Description

### BACKGROUND OF THE INVENTION

### Field of Invention:

The present invention relates to the provision of ordered repeating micro-geometric patterns to bone and tissue interface zones of orthopedic implants, to effect enhanced a direct adhesion to tissue and osseointegration of an implant to bone.

### Prior Art

Numerous publications establish that cell attachment growth, migration and orientation, as well as extracellular matrix synthesis and orientation thereof, are moderated by substrate surface shape (i.e., microgeometry) as well as by surface chemistry. However, the findings in such publications do not address the effect different substrate microgeometrics and dimension would have on various cell colonies' growth and migration parameters as opposed to the morphology of individual cells. Thus, while the prior art establishes that surface microgeometry of substrates influences cell orientation, it does not disclose or suggest what effect different surface microgeometry as implants would have on either the rate or direction of the cell colony growth of different cells of soft tissue or bone surrounding or abutting such a substrate.

Surface microgeometry interaction between soft tissue or bone and implant surfaces has been demonstrated on ceramic and metallic orthopedic implants. This interaction indicates that smooth implant surfaces promote the formation of thick fibrosis tissue encapsulation and that rough implant surfaces promote the formation of thinner, soft tissue encapsulation and more intimate bone integration. Smooth and porous titanium and titanium alloy implant surfaces have been shown to have different effects on the orientation of fibrous tissue or bone cells in vitro. In addition, surface roughness was demonstrated to be a factor in tissue or bone integration into implants having hydroxyapatite surfaces and to alter the dynamics of cell attachment and growth on polymer implants whose surfaces had been roughened by hydrolytic etching.

From the examination of in vitro growth characteristics of cells cultured on flat surfaces there have evolved the following cell "behavioral" characteristics:
1. attachment-dependent growth: the dependence of normal diploid cell or substrate attachment for normal growth;
2. density-dependent inhibition: the tendency of such cells to slow or stop growing once a confluent monolayer is formed;
3. substrate-exploring function: the ability of some types of cells to migrate on a surface in search of acceptable areas for attachment and growth; and
4. contact guidance: the ability of some types of cells to migrate and orient along physical structures. See J. L. Ricci, et al Trans.Soc.Biomat.. 17.253 (1991); J.L.Ricci, et al, Tissue-Inducing Biomaterials, Mat. Res. Soc. Symp. Proc. 252,221-229 (1992); J.Ricci, et al., Bull.Hosp. Joint. Dis. Orthop. Inst. supra; J.L. Ricci, et al, J. Biomed Mater Res. 25(5), supra.; D.M. Brunette, et al, J. Dent. Res., 11-26 (1986); P. Clark, et al. Development, 108, 635-644 (1990).

The behavioral characteristic of cellular contact guidance has been demonstrated in vitro on a variety of surfaces such as grooved titanium, grooved epoxy polymer, and collagen matrix materials of different textures and orientations. Grooved machined metal and polymer surfaces have also been shown to cause cellular and extracellular matrix orientation in vivo and to encourage or impede epithelial downgrowth in experimental dental implants. B. Cheroudi, et al. J Biomat. Mater. Res. 24. 1067-1085 (1990) and 22. 459-473 (1988); G.A. Dunn, et al supra; J. Overton, supra; S.L. Shor. supra; R. Sarber, et al, supra.

Substrates containing grooves of different configurations and sizes have been shown to have orientating effects on fibroblasts and substrates containing grooves of varying depth have been shown to have different degrees of effect on individual cell orientation establishing that grooved surfaces can modulate cell orientation in vitro and can cause oriented cell and tissue growth in vivo. For example, it has been shown that fibrous tissue forms strong interdigitations with relatively large grooves in the range of about 140 µm and can result in an effective barrier against soft tissue downgrowth perpendicular to the grooves. It has also been shown that smaller grooves on the order of about 3-22 µm were more effective in the contact guidance of individual cells. D.M. Brunette, et al. Development, supra; P. Clark et al, supra.

The findings in these publications do not address what effects different substrate microgeometries and sizes would have on various cell colonies growth and migration parameters as opposed to morphology of individual cells. That is, these publications do not disclose or suggest what effect different surface microgeometry of implants would have on either the rate or direction of the cell colony growth of different cells and different tissues surrounding an implant. In addition, these publications do not disclose or consider the most effective textured substrate or crude microgeometry for controlling cell colony growth.

The current methods used to texture the surfaces of implants typically employ sand, glass bead and alumina grit blasting techniques, and acid etching techniques, of the implant surface. In sand, glass bead or alumina grit blasting techniques, compressed air is generally used to drive a blasting medium onto the implant surface at a high velocity to deform and, in some instances, remove portions of the implant surface. The surface texture obtained depends upon the size, shape and hardness of the implant material and on the velocity at which the blasting medium is driven onto the implant surface. The most common surfaces produced by sand or glass bead blasting are matte or satin-finish, while alumina grit blasting produces a random roughened surface.

In acid etching techniques a pattern or mask is placed upon that surface of the implant desired not to be texturized. The exposed parts are then typically treated with an acid that corrodes the exposed surface of the implant whereupon the acid treated surface is washed or neutralized with an appropriate solvent and the pattern or mask is removed

Illustrative of the sand or glass bead blasting technique is the method disclosed in U.S. Pat. No. 5,057,208 to H.R. Sherry, et al wherein the implant surface is shot blasted with metal shot followed by glass bead blasting and then electropolishing.

Illustrative of an acid etching technique is the method disclosed in U.S. Pat. No. 4,778.469 to R.Y. Lin, et al wherein an acid soluble (e.g., aluminum or zinc) space occupier is used. The space occupier contains the pattern to be transferred to the implant surface and is placed on the desired portion of the implant surface that is to be texturized. The space occupier is pressed into the implant surface and is then removed by treating it with acid.

It has been found that these typical blasting techniques leave debris from the processing materials embedded in the implant surface as contaminants. This debris has also been found in soft tissue isolated from the areas adjacent to failed press-fit total hip replacements indicating that the debris was released from the surface of the implants. These problems of residual contaminants debris have been overcome by using the use of laser systems which produces texturized microgeometric substrates without introducing embedded, particulate contaminants. See, for example, U.S.P.N. 5,645,740 and 5,607,607 to Naiman and Lamson. This instant invention refines and extends the teaching thereof with particular reference to orthopedic implants. The prior art is also characterized by implants intended for use in soft tissue, such as U.S. Patent No. 5,011,494 to Von Recum, et al and its related patent family. Therein, texturized surfaces of implants are provided with a variety of geometric configurations which comprise a plurality of projection and recesses formed in a three-dimensional body. It is therein specified that the mean bridging, breadth and diametric distances and dimension play a role in optimizing cell anchorage to implant surfaces. However, the teaching of Von Recum is not applicable to hard bone-like organic tissue, as exists in an orthopedic environment.

Another reference which employs randomized roughing of an implant is U.S. Patent No. 5,571,017 (1996) to Niznick, does not address the orthopedic area and does not employ an ordered or pre-established repetitive micro-geometric surface pattern. Similarly, U.S. Patent No. 4,320,891 (1982) to Branemark employs a randomly micro-pitted surface to create pores in a range of 10 to 1000 nanometers (one micrometer). See Fig. 36. Further, Branemark states that the optimal results in his system are obtained with pore diameters equal to or smaller than about 300 nanometers. Therein, although Branemark indicates that his implant surfaces may assume a pattern of grooves, corrugations or channels, such geometrics are not ordered or repetitive, and it is apparent that the range of focus thereof is in the range of 0.3 to 1 micron in terms of diameters or width of such structures, whereas the lowest end of these invention relate to alternating ridges and grooves having a minimum width of six microns and extending in width to about 15 microns, the same based upon clinical studies as are more fully set forth below. Further, based upon the much smaller surface dimensions with which Branemark is concerned, it is clear that the focus of Branemark is that of individual cell growth, this as opposed to promotion of rate, orientation and direction of entire colonies of cells, i.e., the object of the present invention.

U.S. Patent No. 4,553.272 (1985) to Mears relates, as in Van Recum above, to the development of porous implants having pore sizes in a range of 25 to 400 microns, that is, a minimum range which is well in excess of the maximum range applicable to the ordered microgeometric repetitive surface patterns taught herein. Also, in view of the large dimension of the channels taught by Mears, no relationship exists or is suggested between cell size, structure size, and cellular control resultant thereof.

U.S. Patent No. 5,004,475 (1991) to Vermeire relates to a hip prosthesis having channels or grooves which, similarly, to Mears, are intended to promote tissue ingrowth but which do not correlate between surface microgeometry, cell size, and cell growth. Further Vermeire does not teach any preferred structure or dimension for the channels or grooves thereof.

Thereby, all prior art of record addresses the issue of bone adhesion to an implant at either a level of tissue ingrowth entailing a dimension well above that set forth herein or relates to control of the growth or orientation of individual cells, as opposed to cell colonies, which resultingly require employment of surfache characteristics of dimensions substantially smaller than that employment by the within inventors.

WO 95/12369 A1 describes an orthopedic implant according to the preamble of claim 1. Furthermore, this document describes the growth of bone cells or soft tissue cells on a surface.

### SUMMARY OF THE INVENTION

The invention relates to an orthopedic implant system comprising implant element for surgical insertion into a bone, replacing a joint of a patient, the surface of the having an ordered microgeometric repetitive surface pattern in the form or a multiplicity of alternating ridges and grooves, each having a fixed or established width in a range of about 2.0 to about 25 microns (micrometers) and a fixed or established depth in a range of about 2 to about 25 microns, in which said microgemoetric repetitive patterns define a guide for preferential promotion of the rate, orientation and direction of growth colonies of cells of said bone, which is in contact with said surface pattern. Such an implant may also include a contact zone for soft tissue content.

It is accordingly an object of the invention to provide microgeometic surfaces which alter the growth behavior of colonies of bone-related cells attached thereto.

It is another object to provide microgeometric surfaces of the above type having cross-sectional configurations, which are preferential to particular cell or tissue types.

It is a further object to provide microgeometric implant substrate for controlling in vivo cell attachment, orientation growth, migration and tissue function and therein having dimensions preferential for the prevention of cell growth in a first-axis and for the inducement of growth along a second axis.

It is a further object to provide repetitive microgeometric texturized configurations to implants applicable in a variety of orthopedic applications.

The above and yet other objects and advantages will become apparent from the hereinafter-set forth Brief Description of the Drawings, Detailed Description of the Invention and Claims appended herewith.

### BRIEF DESCRIPTION OF THE DRAWINGS

Fig. 1 is a plan diagrammatic view in an xy axis and at about 750 magnifications, showing ordered microgemetric surface patterns having parallel ridges and grooves, each of approximately equal width, in accordance with the present invention.
Fig. 2 is a view, similar to that of Fig. 1, however in which successive y-axis width of said ridges and grooves vary with y-axis direction of the surface pattern thereof.
Fig. 3 is a diagrammatic plan view of an ordered microgeometric surface pattern which defines a bi-axial x-y matrix formed of alternating recesses and projections along each axis.
Fig. 4 is a plan view, similar to that of Fig. 3, however showing a pattern in which all recesses and projections thereof are co-linear with each other.
Fig. 5 is a plan view, similar to that of Fig. 4, in which all ridges are circular in x-y cross-section.
Fig. 6 is a view, similar to that of Figs. 3 thru 5, in which the grooves of the pattern define an xy grid as the surface pattern thereof.
Figs. 7 thru 14 are yz plane cross-sectional views of the patterns of Figs. 1 thru 6 showing variations in yz plane geometry, that is, relationship of grooves to ridges that are applicable to one or more of the xy plane patterns shown in Figs. 1 thru 6.
Fig. 14A is a 700 power micrograph showing an implant surface formed in accordance with the pattern of Fig. 14.
Figs. 15 thru 19 show further xy plane surface patterns which do not belong to the invention and, respectively, comprise radiating, concentric, circular, radiating fan, radiating with concentric, and radiating with intersecting polar, patterns.
Fig. 20 is a conceptual view of a hip implant provided with orthogonal microgeometric surface pattern of the type of Fig. 1. They are not diagonally oriented as defined in claim 1.
Fig. 21 shows a bar graph that demonstrates bone penetration into channels of the canine ingrowth chamber.
Fig. 22 is a cross-sectional schematic view showing ingrowth into MG-lined and alloy lined micro channels of an orthopedic splint further to Fig. 21
Fig. 23 is a micrograph showing a first instance of bone trabeculae attaching generally parallel to the microgrooves of an MG12 surface of the instant invention.
Fig. 24 is a micrograph showing a second instance of bone trabeculae attaching generally-parallel to the microgrooves of an MG12 surface of the instant invention.
Fig. 25 is a micrograph showing a first instance of extensive bone coverage and numerous fragments of fractured bone remaining in the microgrooves of the MG12 surface of the instant invention; and
Fig. 26 is a micrograph showing a second instance of extensive bone coverage and numerous fragments of fractured bone remaining in the microgrooves of the MG12 surface of the instant invention.
Figs. 27 and 28 are conceptual views of orthopedic pins furnished with microgrooved surfaces. The embodiment of Fig. 27 is not oriented diagonally relative to a mayor axis of the implant.

### DETAILED DESCRIPTION OF THE INVENTION

### BONE STRUCTURE

Bone tissue is the rigid supporting tissue constituting the principal component of all-adult vertebrate skeletal structures. It exists in either dense or spongy form, known respectively as compact and cancellous bone. The typical bone cell size is of the order of about 10 microns.

Bone tissue consists of a chemical mixture of inorganic salts (65 to 70 percent) and various organic substances (30 to 35 percent) and is both hard and elastic. Its hardness is derived from inorganic constituents, principally calcium phosphate and calcium carbonate, with small amounts of fluorides, sulfates, and chlorides; its elasticity is derived from such organic substances as collagen, elastic cellular material, and fats. Internal tubular structures called Haversian canals contain nerve tissues and blood vessels that provide bones with organic nourishment. Surrounding these canals is a somewhat porous tissue composed of thin plates, known as lamellae, and usually containing cavities filled with a network of connective tissue called marrow or myeloid tissue. Bone marrow accounts for from 2 to 5 percent of the body weight of a person and consists of tissue of two types. Yellow bone marrow is made up principally of fat, and red bone marrow is tissue in which red and white blood cells and blood platelets originate. The external portions of bones, enclosing all the components mentioned above, include the compact and hardest of all bone tissue, which is in turn generally sheathed by a vascular, fibrous membrane known as the periosteum.

### MICRO-TEXTURING OF SURFACE

With respect to bone and soft tissue adhering thereto, it has been found that the rate and direction of cell colony growth and the growth of different cell types surrounding a surgical implant can be controlled and effected by using the implants of this invention. In general, such implants comprise a plurality of separate zones of textured surface, each zone containing a different repetitive microgeometric design or pattern which is presented and exposed to the particular cell type for development of its unique colony growth. These different repetitive microgeometric textured design surfaces are intended to:
(a) promote the rate and orient the direction of bone growth, and discourage the growth of soft tissue to achieve secure fixation of the implant surface to bone tissue;
(b) promote the rate and orient the direction of the growth of soft tissue while discouraging the growth of bone tissue to achieve soft tissue integration with the implant surface; and/or
(c) create a barrier that discourages the growth of soft tissue, particularly soft fibrous tissue, and thereby prevent the migration of soft tissue growth in bone tissue attachment surfaces of the implant.

The implants of the invention can be provided from suitable and acceptable materials that are commercially available such as cast or wrought cobalt and chrome alloys, various grades of commercial titanium, titanium alloys, stainless steel alloys, thermoplastic resins such as polyethyletherketone, ceramics, alumina, as well as combinations thereof.

### EXAMPLE OF ENHANCED BONE GROWTH SURFACE TO DIRECT CONDUCTION OF BONE TISSUE

A surface consisting of 12µm grooves and ridges has been shown to increase the RBM (rat bone marrow) to RTF (rat tendon fibroblast) cell colony growth ratio to encourage bone cell growth over fibrous tissue growth. In addition, this surface caused specific directional migration of bone cells at approximately twice the rate of cells on a flat surface. In vivo canine experiments refined implant designs that combine three different types of microgeometries; a surface to enhance bone growth and discourage soft tissue growth, thereby achieving optimal bony fixation; a second surface that encourages soft tissue growth and mitigates against bone growth, to achieve soft tissue integration; and a third "barrier" surface to prevent soft tissue migration into bone attachment areas. In one experiment, data obtained from multichannel implant chambers were used to derive optimal micro-configurations. The ability of these micorgeometries to work in combination was then tested in a second experiment utilizing a customized canine intramedullary femur implant. These surfaces achieved optimal results by selectively encouraging and discouraging site-specific tissue ingrowth. They can be used to enhance bone versus soft tissue growth as well as to direct bone growth into regions of an implant surface where bone fixation is needed.

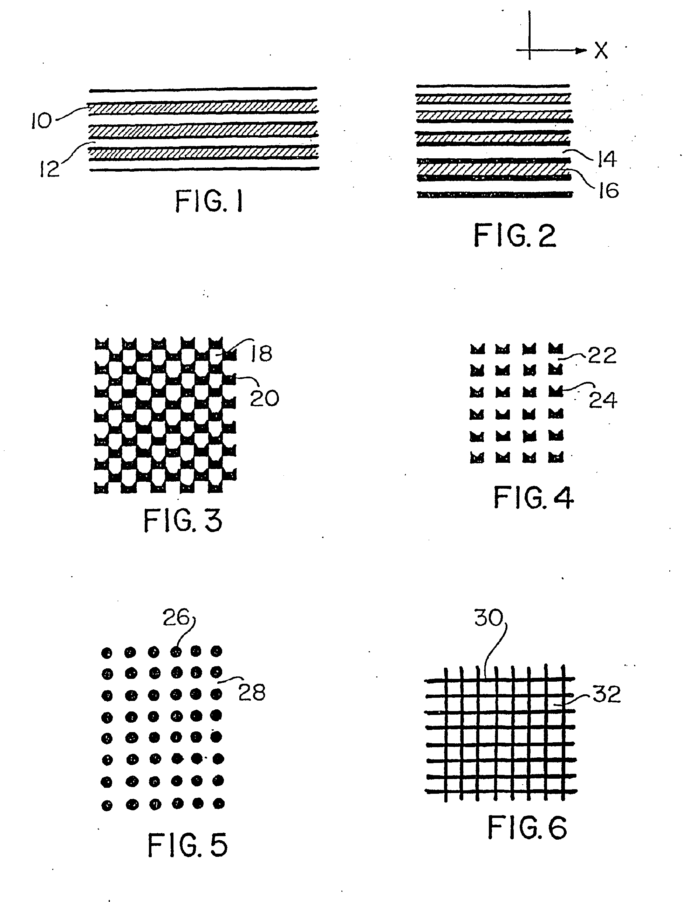

With reference to the means by which the above set forth principles and experimental data may be reduced to practice, it has been found that orthopedic implants may be selectably surfaced in the fashion illustrated in Figs. 1 thru 6 which show a variety of patterns which may comprise ordered microgeometric, repetitive surface patterns, and which may be applied to materials inclusive of titanium, stainless steel, plastics, ceramics, biocompatible glass and combinations thereof which materials may be coated with coatings inclusive hydroxyapatite, RBM roughening, titanium, plama sprayed, calcium sulfate, biocompatible glass, collagen, growth factor compounds, and combination thereof. More particularly, with reference to Fig. 1, the subject ordered microgeometric repetitive patterns may take the form of a multiplicity of alternating grooves 10 and ridges 12 in which each respective ridge and groove displays a width between about 6.0 to about 25 microns and a depth in a range between about 2 to about 25 microns. In the embodiment of Fig. 1, an infinite repeating pattern of co-parallel linear ridges and grooves having substantially equal width defines a micro textured surface of an implant or substrate as contemplated by the instant invention.

In the embodiment of Fig. 2 is shown a surface in which alternating ridges 14 and grooves 16 increase y-axis in width with reference to a transverse axis relative to the axis of said ridges and grooves. Accordingly, with reference to types of tissues with which a transition of tissue type or gradient of tissue density exists, a textured surface of the type of Fig. 2 may be employed.

In Fig. 3, is shown a surface pattern in which ridges 18 take the form of projections while grooves 20 take the form of recesses to thereby define a checkerboard configuration. Therein such ridges and grooves alternate with reference to both a x and y axes of a given surface.

The embodiment of Fig. 4 differs from that of Fig. 3 in that ridges 22 thereof form a bi-axial linear pattern. Similarly, grooves 24 of the embodiment of Fig. 4 define a x-y matrix formed of recesses that may assume a number of geometrics.

In Fig. 5 is shown embodiment of the invention in which circular depressions 26 define grooves or depressions while the areas therebetween, namely, spaces 28 define ridges or projections. It may, therefrom be appreciated that the terminology "alternating ridges and grooves," as used herein, encompasses a variety of microtexturized geometric patterns in which the ridges and grooves thereof while alternating relative to each other may themselves comprise any one of a variety of geometries inclusive of channels, rectangles, parallelograms, squares, circles and ovals.

With reference to Fig. 6, there is shown a grid like arrangement in which grooves 30 define an xy matrix which is etched into a surface 32 such that surface 32, when viewed relative to etched grooves 30, comprises ridges It is believed that this arrangement possesses particular utility in the area of orthopedic implants, as is set forth below.

From the embodiment of Figs. 1 thru 6 it may be appreciated that the width (or diameter) of a given groove need not correspond to that of its respective ridge, providing such widths fall within the above-referenced range of about 2 to 25 microns with a depth in a range of about 2 to about 25 microns. It has, thereby, through extensive experimentation as set forth above, been determined that a micro-geometric repetitive pattern within the scope of the present invention may define a guide for preferential promotion of the rate, orientation and directionality of growth of colonies of cells of bone without requirement that the width of a ridge be equal to that of a groove in that it is, essentially, the groove of the microtexturized surface that defines the guide for preferential promotion of growth of colonies of cells. In most applications, it is desirable to maximize the density of grooves upon a given surface to thereby attain the desired cell growth effect; however, differing clinical environments will dictate use of different surface patterns and density if distribution of grooves.

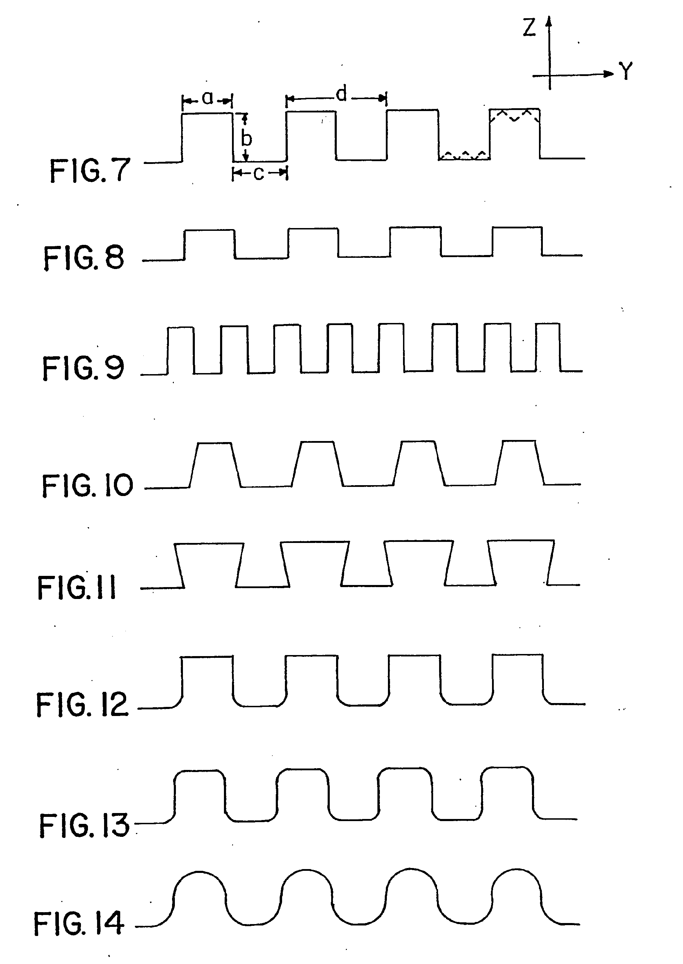

With reference to the views of Figs. 7 thru 14, there is shown diagrammatic cross-sections which may be employed in association with the microgeometric textured configurations above described with reference to Figs. 1 thru 6. In other words, the views of Figs. 7 thru 14 illustrate the range of geometries which may be defined within the yz plane of the surface patterns. Resultingly, Figs. 7 thru 9 show variations in ridge width a, ridge and groove height b, and groove width c. Typically, ridge height will equal groove depth. Parameter d is the sum of ridge and groove width.

The right side of Fig. 7 indicates that y-axis surfaces need not be linear.

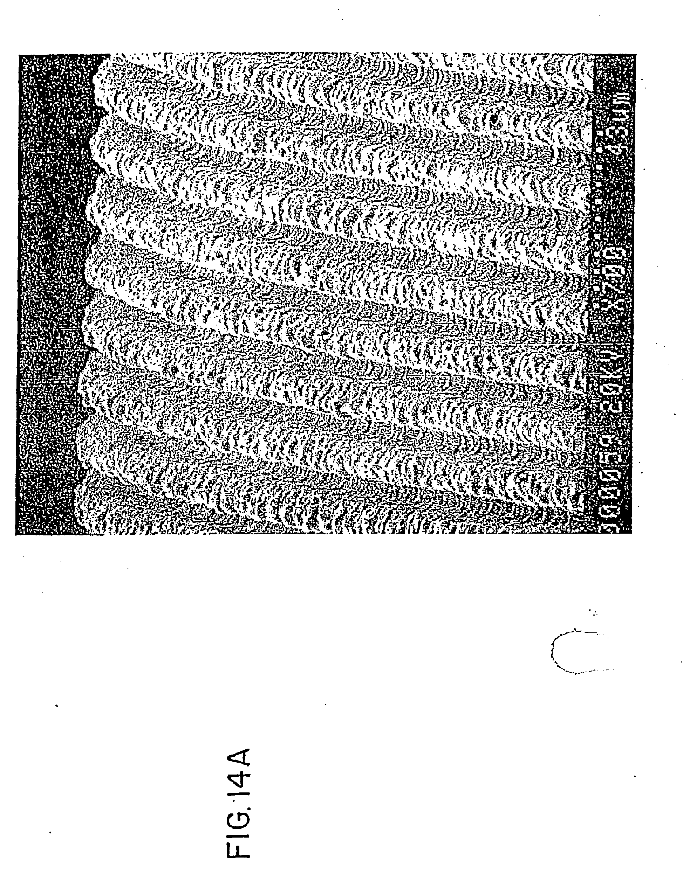

Figs. 10 and 11 show that the walls of the ridges and/or grooves may be yz sloped either inwardly or outwardly relative to the z-axis. Figs. 12 and 13 show that the transition from a y-axis ridge surface to a groove surface need not be a sharp one but, rather, may be curved. In Fig. 14 is shown a cross-sectional view of a pattern in which all yz surfaces are sinusoidal. In such embodiment, the potential for "bridging" between lines of cell colony growth is maximized since an abrupt delineation between ridges and grooves is not present. A micrograph of an actual implant surface showing the embodiment of Fig. 14 is shown in Fig. 14A.

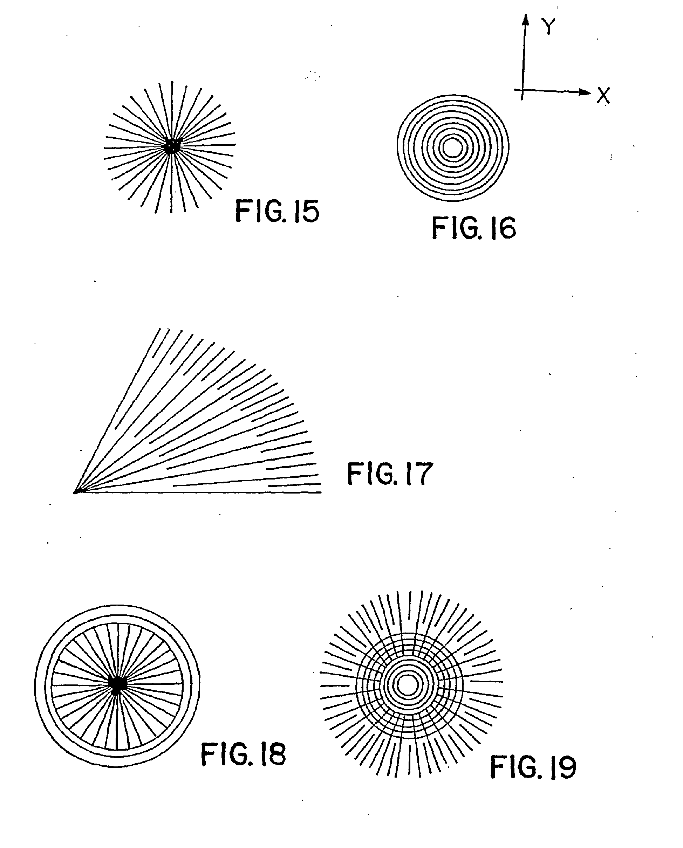

Shown in Figs. 15 thru 19 are exemplary xy surface patterns which are programmable through the use of processes selected from the process group consisting of laser etching, acid etching, mechanical etching and photolithography. More particularly, Fig. 15 comprises a radiating pattern. Fig. 16 a concentric circular pattern, Fig. 17 a radiating fan pattern, Fig. 18 a radiating/concentric pattern, and Fig. 19 a radiating pattern with an intersecting polar pattern.

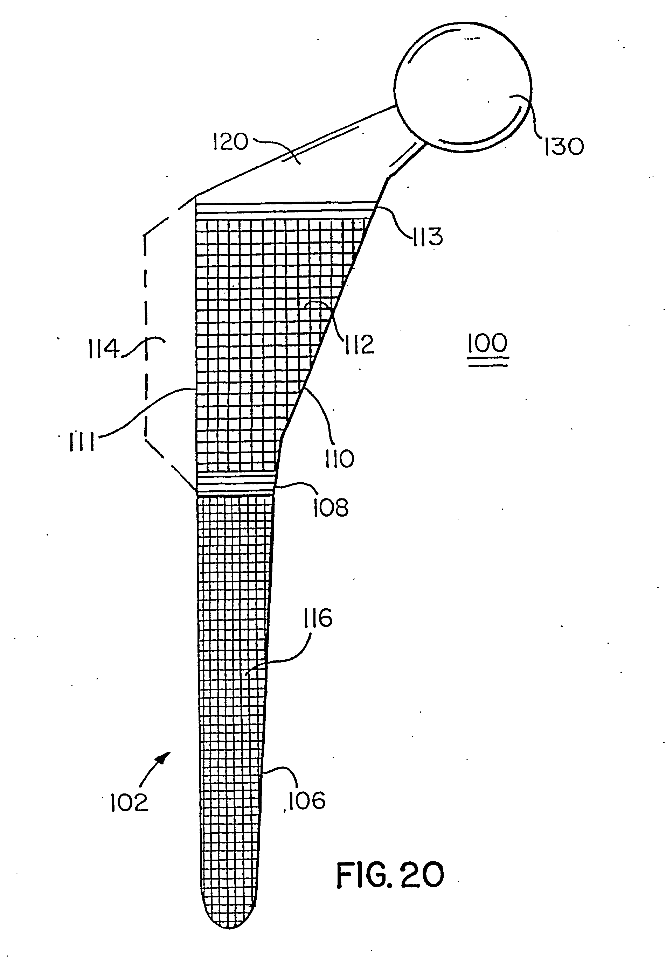

A typical hip implant 102 for humans having microgeometric texturized surfaces is illustrated in Fig. 20. As shown in Fig. 20 the implant comprises a femoral head 130, a femoral neck 120, a proximal stem 110 and a distal stem 106. Proximal stem 110 and distal stem 106 can each be ablated to create microgeometric texturized surfaces 112 and 116 of different geometric designs of patterns in order to enhance and promote the growth of a particular tissue. Premised upon the concept that oriented cells produce oriented tissue, selected portions on surface of the implant are not ablated to prevent migration of tissue growth from one part of the implant to another.

Thus, four separate zones, i.e., zones 108, 112, 113, and 116 are created on the implant surface. Zone 106 corresponds to the texturized surface on the proximal stem 110, zone 106 corresponds to the texturized surface 116 on the distal stem 106, and zones 108 and 113 represent barrier surfaces. The texturized surface in zone 106 on the proximal stem 110 is patterned to promote the rate and orient the direction of the growth of bone tissue on proximal stem 110 and the texturized surface in zone , 106 on the distal stem 106 is patterned to promote the rate and orient the direction of the growth of soft tissue on the distal stem 106. Barrier zone 108 is provided to prevent migration of soft tissue growth into zone 112 of the proximal stem 110 and prevent migration of bone tissue into zone 116 of the distal stem 106. Barrier zone 113 is provided to prevent migration of soft tissue from the femoral neck 120 into zone 112 of the proximal stem 110.

Where excessive stress is anticipated to be exerted on the lateral region 111 of the hip implant, it may be desirable to provide a reinforced hip implant where the lateral region 111 has been built up as indicated in dashed lines 114 to impart additional strength to the implant. In either instance, the lateral region 111 or a built up lateral region 114 is preferably not ablated so as to act as an additional barrier zone in preventing migration of bone tissue growth from the proximal stem 110 into either lateral region 111 or built up lateral region 114.

Implant surfaces ablated to have individual zones of different microgeometric texturized designs or patterns separated from one another by barrier zones provide implants that have good contact in the medial and lateral regions, but not in the anterior and posterior regions; and, achieve bone tissue fixation in the proximal region while preventing its migration into the bone tissue attachment regions.

### MICROGEOMETRY SURFACE TESTING IN THE CANINE CHAMBER MODEL

The MG12 surfaces of the present invention were compared with industry standard aluminum-grit-blasted cpTi ("GCP") and Ti-alloy ("GA") surfaces in the canine chamber model. The microgrooved surfaces showed significantly greater bone ingrowth and apposition than either of the industry-standard roughened surfaces. The MG12 surfaces exhibited such intimate bone apposition than direct bone attachment could be demonstrated using tensile testing of these samples. Further experiments using the same model were run comparing MG8 surfaces to the MG12 surfaces. These surfaces were also observed to control the geometry of the attached bone. While the normal ingrowth pattern of trabecular in this model is random, the MG12 surfaces showed orientation of attached bone trabeculae in a direction parallel to the microgrooves. The results of these experiments are significant for their demonstration of direct bone attachment to a laser microtextured metal implant surface, and the affinity of implant surface microgeometry to control the architecture of bone attached to its surface.

### IN VIVO RESPONSE TO MACHINED SURFACES

Canine Chamber studies were conducted comparing bone and soft tissue response to MG12. GCP, and GA surfaces, at 4, 6, and 12 weeks, respectively. Faxitron (high resolution x-ray) morphometry, electron microscopy, and medical testing were employed in the studies.

### BONE INGROWTH AND APPOSITION

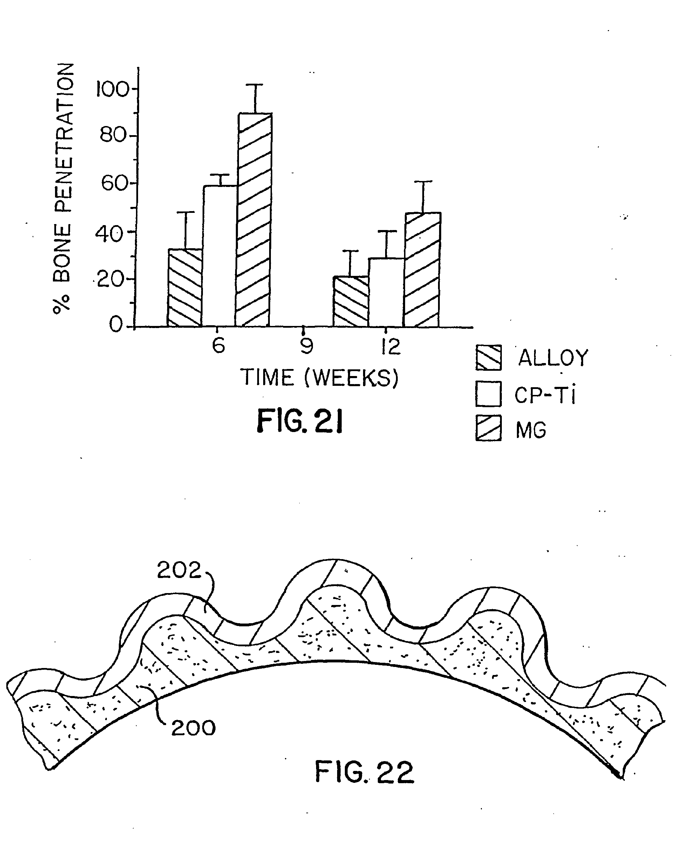

Faxitron morphometry showed significantly larger amounts of bone penetration into channels lined with the MG12 surfaces, at 6 and 12 week, respectively, versus the GCP and GA surfaces (see Fig. 21). This bone ingrowth exhibited close apposition to the MG12surfaces lining the channels. Ingrown bone did not exhibit this close apposition with the GCP or GA surfaces (see Fig. 22). Fig. 21 shows the percentage of bone ingrowth into channels lined with grib-blasted alloy, grit-blasted CP-titanium, and micorgrooved CP-titanium at 6 and 12 weeks. At both points, MG surfaces showed significantly more ingrowth. Fig. 22 is a cross-sectional schematic view of a chamber having bone ingrowth 200 into MG-lined 202 channels and alloy-lined channels. Bone penetration and apposition to metal surface are significantly greater on the MG surfaces.

### MECHANICAL TESTING

After close bone apposition to the MG12 surfaces was observed, a mechanical testing protocol that is normally used for testing of bone attachment to plasma-sprayed-hydroxyapatite-coated samples, was instituted. This protocol involves tensile testing of the individual samples removed from the chambers, by fixing the parallel plates of each sample in a servo-hydraulic testing system. The samples are thereafter tested in increasing tension until they fail. The data are expressed as force to failure in Newtons. The tested interface represents a 5 mm x 8 mm surface. Thus, assuming complete bone covering of the interface, 40 N represents 1 Mpa of failure strength. However, this is a conservative estimate since complete bone coverage is seldom achieved.

At 6 week, mechanical testing of these specimens showed no significant bone attachment to the GCP or GA samples. These sample simply fell apart when removed from the implantable chambers. The MG12 samples showed rigid bone attachment when removed from the implantable chambers. The MG12 samples showed rigid bone attachment when the samples were removed from the chamber, and failed in tension at an average of 12.5 (±9.8) N of force to failure. These results indicate that MG12 surfaces do not show significant fibrous tissue growth and, instead, show early direct bone attachment. This result does not occur when standard roughened surfaces are used.

### ELECTRON MICROSCOPY OF THE BONE/MG12 INTERFACE

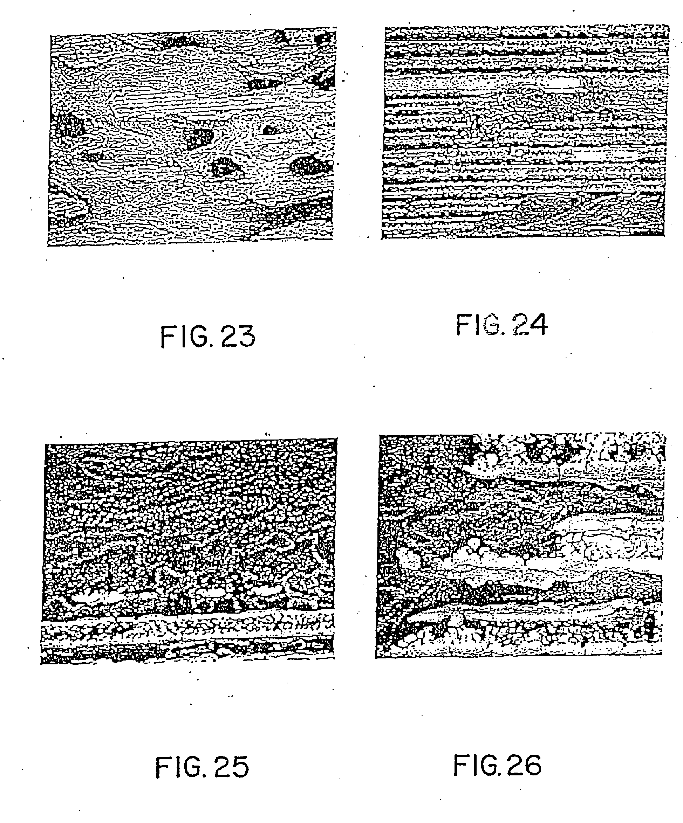

The failed mechanical tested specimens were first deproteinated to remove soft tissue. When thereafter scanned with electron microscopy, the specimens showed extensive, direct bone attachment to the MG12 surface. As shown in Fig. 23, the impression of the MG12 surface remained in the bone pulled from the implant surface. As shown in Fig. 24, individual bone trabeculae were left attached to some areas the MG12 surface indicating that around some areas of the surfaces, bone/bone interface failed before the bone/implant interface. Examination of the tested MG12 surfaces showed extensive bone coverage and numerous fragments of fractured bone left behind in the surface microgrooves and interlock with the surface. Only small isolated areas of direct bone apposition were observed on the GCP and GA surfaces.

In addition, as shown in both Fig. 23 and Fig. 24, the trabecular structure of the bone attached to the MG12 surfaces was observed to follow the surface microstructure. Moreover, as shown in Fig. 23 and Fig. 26, attached bone trabeculae were observed to be predominantly oriented parallel to the surface microgrooves.

Bone response to GCP GA, and MG12 surfaces thereby showed that the MG12 surface exhibited a superior bone interface when compared with grit roughened surfaces widely used in the orthopedic implant industry. The MG12 surface showed more extensive bone penetration and less fibrous tissue interface. In addition, the MG12 surface exhibited direct bone bonding, as result that was not seen in the roughened surfaces. The MG12 surface also exhibited an ability to control the architecture of the attached bone. These results are significant because they demonstrate that the surface of a permanent metal implant may be surface possessed to achieve direct bone attachment, and that this surface microgeometry can be used to control microarchitecture at the bone/implant interface.

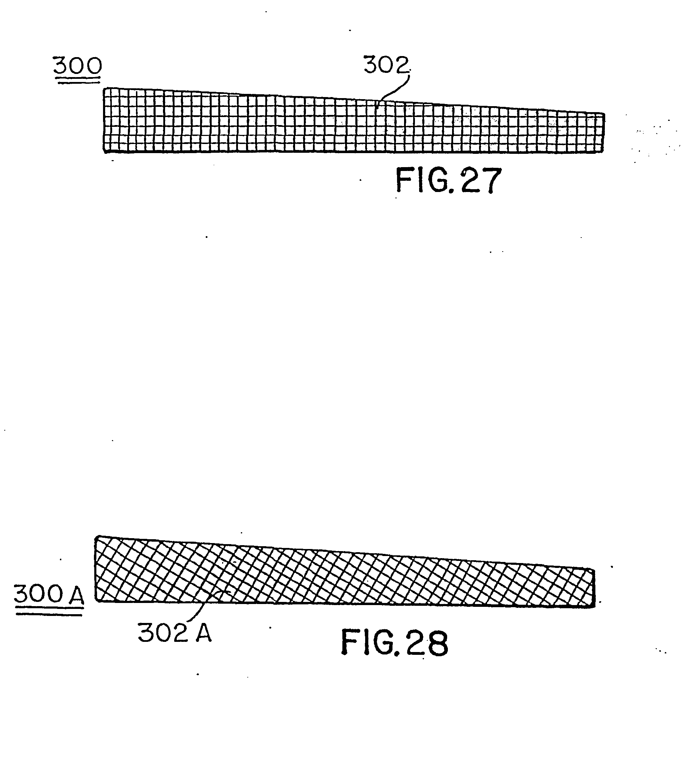

Shown in Figs. 27 and 28 are orthopedic pins furnished with microgroove surface.

While there has been shown and described the preferred embodiment of the instant invention it is to be appreciated that the invention may be embodied otherwise than is herein specifically shown and described and that, within said embodiment, certain changes may be made in the form and arrangement of the parts without departing from the underlying ideas or principles of this invention as set forth in the Claims appended herewith.

## Claims

1. An orthopedic implant (300A) comprising an implant element for surgical insertion into a bone or bone-related tissue of a patient, said implant element comprising an ordered microgeometric, repetitive surface pattern (302A) in a form of a multiplicity of substantially parallel alternating ridges (12,14,18,22,28) and grooves (10,16,20,24,26), each having an established width in a range of about 2 to about 25 microns, and an established depth in a range of about 2 to about 25 microns, said implant element comprising an orthonormal matrix of said pattern of alternating ridges (12,14,18,22,28) and grooves (10,16,20,24,26), said micro-geometric repetitive pattem defining a guide for a preferential promotion of the rate, orientation and direction of growth of colonies of cells of said bone which are in contact with said surface pattem (302A),
**characterized in that**
the orthonormal matrix is oriented diagonally relative to a major axis of the implant,

2. The implant as recited in Claim 1 in which base materials of said implant are selected from the group consisting of the materials of titanium and alloys thereof, stainless steel, ceramics, biocompatible glass and combinations thereof.

3. The implant as recited in Claim 2 in which a surface of said implant element comprises a coating selected from the group of surfaces consisting of hydroxyapatite, RBM roughening, titanium, plasma sprayed, calcium sulfate, biocompatible glass, collagen, growth factor compounds, and combination thereof.

4. The implant as recited in Claim 1 in which said orthopedic implant is selected from the group consisting hip, knee, shoulder, elbow, ankle and finger implants..

5. The implant as recited in Claim 4 in which said implants are selected from the group consisting of bone and soft tissue anchors.

6. The implant as recited in Claim 4 in which said repetitive micro-geometric pattern (302A) comprises a product of the process selected from the process group consisting of laser etching, acid etching, mechanical etching, and photolithography.

7. The implant as recited in claim 4 comprising different zones furnished with respectively different surface patterns.

8. The implant as recited in Claim 7 in which said different zones include respective hard and soft tissue contact zones.

## Patentansprüche

1. Orthopädisches Implantat (300A), welches ein Implantatelement zur chirurgischen Einbringung in einen Knochen oder ein mit einem Knochen verbundenes Gewebe eines Patienten aufweist, wobei das Implantatelement ein geordnetes, mikrogeometrisches, sich wiederholendes Oberflächenmuster (302A) in Form einer Vielzahl von im wesentlichen parallelen, sich abwechselnden Erhebungen (12,14,18, 22,28) und Rillen (10,16,20,24,26) aufweist, welche jeweils eine bestehende Breite in einem Bereich von ungefähr 2 bis ungefähr 25 Mikrometer und eine bestehende Tiefe in einem Bereich von ungefähr 2 bis ungefähr 25 Mikrometer aufweisen, wobei das Implantatelement eine orthonormale Matrix des Musters von sich abwechselnden Erhebungen (12,14,18,22,28) und Rillen (10,16,20,24,26) aufweist, wobei das mikrogeometrische, sich wiederholende Muster eine Führung für eine bevorzugte Förderung des Grads, der Orientierung und der Richtung des Wachstums von Kolonien von Zellen des Knochens festlegt, welche in Kontakt mit dem Oberflächenmuster (302A) sind,
**dadurch gekennzeichnet, dass** die orthonormale Matrix im Bezug auf eine Hauptachse des Implantats diagonal orientiert ist.

2. Implantat nach Anspruch 1, bei welchem Grundmaterialien des Implantats aus der Gruppe ausgewählt sind, die aus den Materialien aus Titan und Legierungen davon, rostfreiem Stahl, Keramiken, biokompatiblem Glas und Kombinationen davon besteht.

3. Implantat nach Anspruch 2, bei welchem eine Oberfläche des Implantatelements eine Beschichtung aufweist, welche aus der Gruppe von Oberflächen ausgewählt ist, die aus Hydroxylapatit, RBM-Aufrauung, Titan, plasmagesprüht, Kalziumsulfat, biokompatiblem Glas, Collagen, Wachstumsfaktorzusammensetzungen und Kombinationen davon besteht.

4. Implantat nach Anspruch 1, bei welchem das orthopädische Implantat aus der Gruppe ausgewählt ist, die aus Hüft-, Knie-, Schulter-, Ellbogen-, Knöchel- und Fingerimplantaten besteht.

5. Implantat nach Anspruch 4, bei welchem die Implantate aus der Gruppe ausgewählt sind, die aus Knochen- und Weichgewebedübeln bestehen.

6. Implantat nach Anspruch 4, bei welchem das sich wiederholende, mikrogeometrische Muster (302A) ein Produkt des Prozesses aufweist, welcher aus der Prozessgruppe ausgewählt ist, die aus Laserätzen, Abbeizen, mechanischem Ätzen und Fotolithografie besteht.

7. Implantat nach Anspruch 4, welches unterschiedliche Zonen aufweist, die mit jeweils unterschiedlichen Oberflächenmustern ausgestattet sind.

8. Implantat nach Anspruch 7, bei welchem die unterschiedlichen Zonen jeweilige Hart- und Weichgewebe-Kontaktzonen beinhalten.

## Revendications

1. Implant orthopédique (300A) comprenant un élément d'implant pour insertion chirurgicale dans un os ou un tissu apparenté à l'os d'un patient, ledit élément d'implant comprenant un motif de surface répétitif microgéométrique ordonné (302A) ayant la forme d'une multiplicité de crêtes (12, 14, 18, 22, 28) et de rainures (10, 16, 20, 24, 26) alternées sensiblement parallèles, ayant chacune une largeur régulière allant de 2 à environ 25 microns, ledit élément d'implant comprenant une matrice ortho-normale dudit motif de crêtes (12, 14, 18, 22, 28) et de rainures (10, 16, 20, 24, 26), ledit motif répétitif microgéométrique définissant un guide pour favoriser de façon préférentielle la vitesse, l'orientation et la direction de croissance de colonies de cellules dudit os qui sont en contact avec ledit motif de surface (302A),
**caractérisé en ce que**
la matrice ortho-normale est orientée en diagonale par rapport à l'axe principal de l'implant.

2. Implant tel qu'exposé dans la revendication 1, dans lequel les matériaux de base dudit implant sont choisis parmi les éléments du groupe formé des matériaux suivants : titane et ses alliages, acier inoxydable, céramiques, verre biocompatible et leurs combinaisons.

3. Implant tel qu'exposé dans la revendication 2, dans lequel une surface dudit élément d'implant comprend un revêtement choisi parmi le groupe de surfaces formé de surfaces d'hydroxyapatite, rugosifiées par projection de matériau résorbable (RBM), de titane, vaporisées sous plasma, de sulfate de calcium, de verre biocompatible, de collagène, de composés de facteur de croissance et de leurs combinaisons.

4. Implant tel qu'exposé dans la revendication 1, dans lequel ledit implant orthopédique est choisi parmi les éléments du groupe formé d'implants de hanche, du genou, de l'épaule, du coude, de la cheville et des doigts.

5. Implant tel qu'exposé dans la revendication 4, dans lequel lesdits implants sont choisis parmi les éléments du groupe formé d'ancrage osseux et d'ancrage sur tissu mou.

6. Implant tel qu'exposé dans la revendication 4 dans lequel ledit motif répétitif microgéométrique (302A) comprend un produit d'un processus choisi parmi les éléments du groupe formé de la gravure au laser, la gravure à l'acide, la gravure mécanique et la photolithographie.

7. Implant tel qu'exposé dans la revendication 4, comprenant des régions différentes pourvues chacune de motifs de surface différents.

8. Implant tel qu'exposé dans la revendication 7, dans lequel lesdites régions différentes comprennent respectivement des régions de contact de tissu dur et de tissu mou.
